# EUROPEAN PATENT APPLICATION

(11) **EP 3 957 278 A1**
(43) Date of publication of application: **23.02.2022**
(21) Application number: 21195080.3
(22) Date of filing: 14.05.2015
(51) Int. Cl.: A61F 2/24

(54) **VALVE STENT AND VALVE REPLACEMENT DEVICE**

(30) Priority: 26.03.2015 CN 201510136304
(62) Divisional of application: 15885936.3
(71) Applicant: Venus MedTech (HangZhou), Inc., Hangzhou, Zhejiang 310052 (CN)
(72) Inventor: ZENG, Frank, Hangzhou Zhejiang, 310000 (CN); LO, Larry, Hangzhou Zhejiang, 310000 (CN); QI, Jess, Hangzhou Zhejiang, 310000 (CN)
(74) Representative: KIPA AB

(57) **Abstract**

A valve stent comprises a tubular supporting net frame (12) and a flared section (1) connected to a corresponding end of the supporting net frame (12). The flared section (1) is connected to all end nodes (9) located at a corresponding side of the supporting net frame (12). A section of the supporting net frame (12) is a transition section (3), and a ratio of an axial length of the transition section (3) before being compressed to an axial length of the transition section (3) after being compressed is 1.

## Description

### FIELD OF THE INVENTION

The present invention relates to the technical field of medical apparatuses, and in particularly to a valve stent used safely and a valve replacement device having the same.

### BACKGROUND

In the prior art, an interventional cardiac valve generally includes a compressible valve stent and a plurality of leaflets, wherein the valve stent has good biological compatibility, and can be positioned at a corresponding cardiac valve location safely, stably, and reliably.

A main body of a valve stent is usually a rhombic unit structure, which is configured to meet the requirement of compressibility. However, the rhombic unit structure may form independent rhombic vertices, and these rhombic vertices existing in isolation are usually sharp. During the use process of the valve stent, there is a risk that the rhombic vertices existing in isolation pierce a sheath.

If the rhombic vertices existing in isolation are gathered at an end portion of the valve stent, the risk of piercing is relatively low. However, in order to be fit for particular structures of different valves of hearts, valve stents used in different positions have different structures.

Taking a pulmonary valve as an example, in order to make the location of the valve at the main pulmonary artery more stable, the valve stent is further provided with a flared section located at a branch location of the main pulmonary artery, that is, the rhombic units structure at the end portion of the valve stent are extended further in an axial direction of the valve stent and expanded in a radial direction of the valve stent, such that this portion is softer and can resiliently match with a blood vessel wall, thereby preventing the blood vessel wall from being stabbed or even pierced. However, the changes of the structure on the end portion may cause some vertices of the rhombic unit structures of the valve stent, which exist in isolation, to be exposed.

For example, a Chinese patent application publication CN102961199A discloses a pulmonary artery valve stent which can prevent displacements. The pulmonary artery valve stent includes a valve suture section, an artificial valve connected with the valve suture section, and a position limiting structure connected with a distal end of the valve suture section. The valve suture section is located in a right ventricular outflow tract or a main body of the pulmonary artery after being released, and a vertex portion of the position limiting structure abuts against an intersecting portion of the main body of the pulmonary artery and a branch of the pulmonary artery after being released, thereby providing an axial limiting function. In this patent document, the valve suture section is formed by a plurality of rhombic structure units; when the distal end of the valve suture section is connected with the position limiting structure, some rhombic vertices of the rhombic structure existing in isolation may remain, and these rhombic vertices existing in isolation are potential safety hazards during the use process.

For another example, a Chinese patent CN101951858B discloses a funnel shaped throttling device. As shown in Fig. 1, the funnel shaped throttling device includes a middle portion having rhombic grid structures, and two end portions of flared shape connected to two ends of the middle portion respectively. The middle rhombic grid structures has a plurality of rhombic vertices 10 existing in isolation, may cause much inconvenience during operation process.

When a valve stent is compressed inwards a sheath pipe, the rhombic vertices existing in isolation will become spines. The spines are prone to stab the sheath pipe when passing through complex and curved anatomic paths in a human body. In the subsequent process of releasing valve, they may also result in too much resistance and pierce the sheath pipe, and thereby cause the valve to be unable to release successfully; in extreme cases, they may stab or even pierce blood vessel walls, and then cause great harm to patients.

### SUMMARY OF THE INVENTION

The present invention provides a valve stent used safely and a valve replacement device with the valve stent, which eliminates rhombic vertices existing in isolation which are present in non-end portions of the valve stent, preventing spines from appearing after the valve stent is compressed while maintaining the mechanical properties of the original structural, and thereby resolving the problem of sheath damaged caused by the spines.

A valve stent used safely comprises a tubular supporting net frame and a flared section connected to a corresponding end of the supporting net frame, and the flared section is connected with all end nodes located at a corresponding side of the supporting net frame.

The supporting net frame of the present invention refers to a portion of the valve stent that is configured to support a prosthesis valve, it is generally tubular, blood flows inside the tubular structure and interacts with the prosthesis valve in the tubular structure. The supporting net frame is not limited to a cylinder extending with a constant diameter, and end portions of the supporting net frame may expand or reduce radially.

At least one end of the supporting net frame is connected with the flared section. An existing valve stent is generally provided with fixed flared sections at both two ends, and the two flared sections form an inflow portion and an outflow portion respectively; the inflow portion and the outflow portion are differentiated according to blood flowing directions, that is, blood enters the valve stent via the inflow portion, passes through the supporting net frame, and leaves the valve stent via the outflow portion.

The supporting net frame has rhombic structures, and all the end nodes at the supporting net frame, namely all the vertices of the rhombic structures adjacent to the flared section, are connected with the flared section to prevent isolated rhombic vertices from appearing at non-end portions of the valve stent, and thus the phenomenon that spines are formed after the valve stent is compressed into the sheath pipe is eliminated.

An end of the valve stent that will be released from the sheath firstly is a precedent release end, the flared section is located at the precedent release end, avoiding the phenomenon that spines pierce the sheath when the sheath is retracted. Even if spines are formed at a posterior release end of the valve stent, as the orientations of the spines are similar to the retracting direction of the sheath pipe, and thus the possibility to cause the phenomenon that the spines pierce the sheath is low. Therefore, preferably, the flaring portion is located at the precedent release end of the valve stent. In the prior art, the outflow portion is generally the precedent release end, and the outflow portion is namely the flared section.

An outer rim of the flaring portion is formed by a plurality of curved supporting bars, end nodes of the supporting net frame that correspond to the locations of the supporting bars are all connected with the supporting bars. The end nodes intersect the supporting bars or are connected with the supporting bars tangentially and intersectingly by guiding bars.

All of the end nodes are connected to the supporting bars directly or by the guiding bars, that is, there is no end node existing in isolation at the supporting net frame, and each end node is connected with at least three linear edges; when the whole valve stent is compressed, the end nodes will not become spines.

Two ends of each supporting bar are connected with respective end nodes of the supporting net frame, a middle portion of the supporting bar extends along an axial direction of the supporting net frame, and a portion of the supporting bar near the end nodes is bent outwards to form a flared structure. Each guiding bar gradually deviates from the supporting net frame along an extending path from a corresponding end node to a corresponding supporting bar.

The guiding bars are curved appropriately, and their curvature degrees are adapted for the curved shapes of the supporting bars, such that the guiding bars and the supporting bars are located on the same smooth curved surface.

Preferably, an included angle between a line connecting two ends of the extending path and an axis of the valve stent is in the range of 0-70 degrees. When the included angle between the extending path and the axis of the valve stent is 0 degree, the length of each guiding bar is the shortest; however, since the guiding bars and the supporting bars are curved, the extending path of each guiding bar is generally not parallel to the axis of the valve stent. The guiding bars need to be converged to the supporting bars, in order to be adapted for the shapes of the supporting bars, it is difficult to form a large included angle between the extending path of each guiding bar and the axis of the valve stent.

Preferably, the included angle between a line connecting the two ends of the extending path and the axis of the valve stent is in the range of 20-60 degrees. Further preferably, the included angle between the line connecting the two ends of the extending path and the axis of the valve stent is in the range of 30-45 degrees.

Each guiding bar is connected with a supporting bar that is nearest to the guiding bar, and an intersection angle formed between the guiding bar and the supporting bar at the junction is an acute angle. Using such a structure is easy to maintain the original mechanical performance.

Every four adjacent end nodes are assigned as a group. In each group of end nodes, two ends of a supporting bar are respectively connected with two end nodes that are located farthest from each other, and each of the two middle end nodes is connected to a corresponding side of a supporting bar by a guiding bar; the two guiding bars do not intersect each other, and the junction between each guiding bar and the supporting bar is substantially located at a middle portion in the axial direction of the flared section.

A section of the supporting net frame is a transition section, a ratio of an axial length of the transition section before being compressed to an axial length of the transition section after being compressed is 1. Since the axial length of the transition section keeps constant before and after being compressed, compared with the rhombic grids generally used in the prior art, the change of the axial length of the supporting net frame before and after being compressed can be reduced.

Preferably, the axial length of the transition section equals at least 40% of a total length of the supporting net frame. Only if a ratio of the axial length of the transition section to the total length of the supporting net frame reaches 40% or more, the effect of the transition section can appear, that is, due to the transition section, a length of a compressed supporting net frame can be reduced so as to meet the requirement of adaptability for bending, such that the supporting net frame can easily reach a predetermined site inside a human body, thereby ensuring that an operation is preformed favorably.

Although the transition section can reduce the change of the axial length of the supporting net frame before and after being compressed, it is not true that the longer the transition section the better. The reason is that, although the axial lengths of the rhombic grids generate great changes before and after being compressed, the structures of the rhombic grids contribute to the strength of the supporting net frame and resists the impact of blood flows for a long time, and the structures of the rhombic grids enable the supporting net frame to be compressed and positioned inside the sheath pipe.

Therefore, preferably, the axial length of the transition section equals 40%-90% of the total length of the supporting net frame. Further preferably, the axial length of the transition section equals 50%-80% of the total length of the supporting net frame.

As the simplest embodiment, the transition section is formed by a plurality of straight rods extending along the axial direction of the valve stent, and the straight rods are evenly distributed along the circumference of the valve stent.

An end portion of each straight rod of the transition section is connected with a corresponding rhombic vertice, and particularly, the end of each straight rod of the transition section is connected with a vertice of a rhombic grid facing the transition section.

The valve stent used safely provided by the present invention is easy to realize, with a production technologies and production efficiency substantially unaffected, it is only required to modify the design drawings of the valve stent and processing laser cutting of the valve stent base on the modified drawings, the manufacturing cost will not increase, all subsequent processing technologies and moulds can use the existing ones, and thus the cost for the improvement is low.

The present invention further provides a valve replacement device, which includes the aforementioned valve stent and a prosthesis valve fixed inside the supporting net frame.

Both the supporting net frame and the inflow portion of the valve stent are covered by films; the prosthesis valve can be sutured onto an inner wall of the valve stent, and can also be mounted and fixed by other known means.

The present invention improves the structures of existing memory alloy self-expandable valve stents, eliminates the vertices existing in isolation and present at non-end portions of the valve stents, preventing spines from appearing after the valve stent is compressed while maintaining the mechanical properties of the original structural, and thereby resolving the problem of sheath damage caused by the spines.

### BRIEF DESCRIPTION OF THE DRAWINGS

Fig. 1 is a schematic view of a valve stent in the prior art;
Fig. 2 is a schematic view showing that an isolated end node pierces a sheath, according to the prior art;
Fig. 3 is a structural schematic view of a valve stent used safely of the present invention (wherein the back side thereof is omitted);
Fig. 4 is a perspective view of the valve stent used safely of the present invention;
Fig. 5 is a structural schematic view of a valve stent used safely according to a second embodiment of the present invention (wherein the back side thereof is omitted).

In the drawings, the corresponding relationship between numbers and components are as follows:
1, outflow portion; 2, second grid portion; 3, transition section; 4, first grid portion; 5, inflow portion; 6, supporting bar; 7, straight rod; 8, guiding bar; 9, end node; 10, end node; 11, sheath; 12, supporting net frame.

### DETAILED DESCRIPTION OF THE PREFERRED EMBODIMENT

The present invention will be further described hereafter with reference to the accompany drawings, taking a pulmonary artery stent as an example.

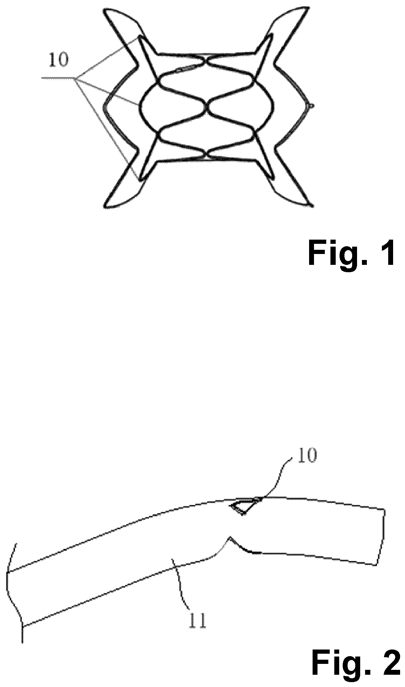

There are end nodes existing in isolation in pulmonary artery stents in the prior art. For example, there are end nodes 10 in a pulmonary artery stent shown in Fig. 1. The end nodes 10 are not located at any end portion of the pulmonary artery stent, after the pulmonary artery stent is compressed into a sheath, the isolated end nodes 10 are prone to be deformed into spines and pierce the sheath 11 during the retracting process of the sheath 11, as shown in Fig. 2.

### Embodiment I

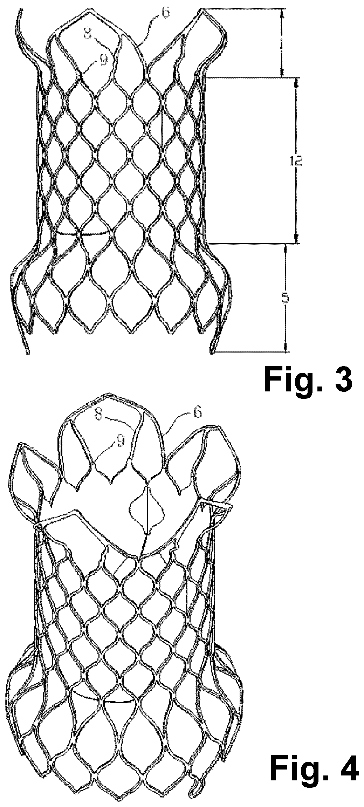

As shown in Fig. 3, the present invention provides a pulmonary artery stent which can be used safely, including a supporting net frame 12, and an inflow portion 5 and an outflow portion 1 that are connected to two axial ends of the supporting net frame 12, respectively.

The inflow portion 5 and the outflow portion 1 expand radially outwardly to form flared sections. When the pulmonary artery stent is released inside a human body, the outflow portion 1 of the pulmonary artery stent is a precedent release end, and the outflow portion 1 is connected to all end nodes 9 at a corresponding side of the supporting net frame 12. A back side of the valve stent is omitted in Fig. 3, and only a front side of the valve stent is shown.

An Outer rims of the outflow portion 1 is formed by a plurality of curved supporting bars 6. Every four adjacent end nodes 9 are assigned as a group, which are connected together by a supporting bar 6 and two guiding bars 8. Two ends of the supporting bar 6 are respectively connected with two end nodes 9 that are the located farthest from each other, each of the two middle end nodes 9 is connected to a corresponding side of the supporting bar 6 by a respective guiding bar 8. The two guiding bars 8 do not intersect each other, and the junction between each guiding bar 8 and the supporting bar 6 is substantially located at an axial middle portion of the outflow portion 1.

Each guiding bar 8 gradually deviates away from the supporting net frame 12 from the corresponding end node 9 to the supporting bar 6. an acute angle is formed between each guiding bar 8 and the supporting bar 6at the junction. An included angle between a line connecting two ends of a respective guiding bar 8 and an axis of the valve stent is 30 degrees.

### Embodiment II

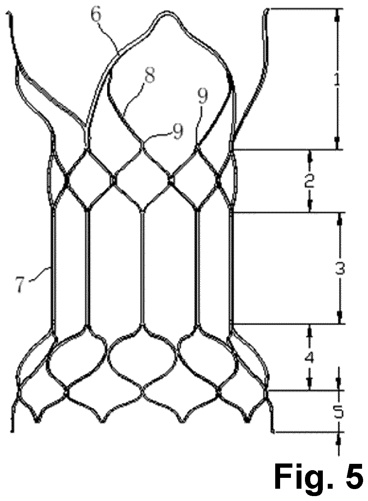

As shown in Fig. 5, the present invention provides another pulmonary artery stent which can be used safely, including a supporting net frame, an inflow portion 5 and an outflow portion 1 connected to two axial ends of the supporting net frame respectively. A middle portion of the supporting net frame is a transition section 3.

One end of the transition section 3 is connected with the inflow portion 5 via a first grid portion 4, and the other end of the transition section 3 is connected with the outflow portion 1 via a second grid portion 2. Both the first grid portion 4 and the second grid portion 2 are formed by continuous rhombic structures. Compared with the transition section 3, the first grid portion 4 expands radially to form a flared section. Rims of each rhombic structure are not exact straight lines but curved outwards slightly, and the number of the rhombic structures of the first grid portion 4 equals the number of the rhombic structures of the second grid portion 2.

The transition section 3 is formed by a plurality of straight rods 7 extending along an axial direction of the valve stent. The straight rods 7 are evenly distributed along a circumference of the valve stent, and an axial length of the transition section is about 45% of a total length of the supporting net frame.

Ends of each straight rod 7 of the transition section 3 are connected with a corresponding rhombic vertice of a rhombic unit, which faces, i.e., located adjacent to, the transition section 3. It can be seen from Fig. 5 that the straight rods 7 of the transition section 3 and the rims of the adjacent rhombic units cooperatively form hexagons, and interior angles of the hexagons are all obtuse angles.

The present invention adopts a design structure of a self-expandable valve stent, which realizes the retraction of the valve stent by the deformation of the rhombic grid section, such that the valve stent can be compressed into a sheath. Moreover, the valve stent can generate a uniform radial supporting force after being implanted into a human body, and thus the valve can be prevented from moving and dropping. As the isolated end nodes are connected to the supporting bars by the guiding bars, the risk that the end nodes pierce the sheath be eliminated, without changing adaptability of the flared section to the blood vessel.

Furthermore, the embodiment II of the present invention can reduce almost 50% of the amount of metal material used by the valve stent (which is usually memory alloy, the present invention uses nickel-titanium alloy), not only reduces a diameter of the valve stent after being compressed, but also improves the adaptability for bending, so that the passing-through performance of the valve in the blood vessel is further enhanced.

The present invention further provides a valve replacement device, which includes an aforementioned valve stent and a prosthesis valve fixed inside the supporting net frame. When the valve stent reaches a predetermined site inside a human body via a transport system, the valve stent is released from a sheath and then expands, and the prosthesis valve fixed inside the valve stent replaces the natural valve of the human body to realize the function of enabling blood to pass unidirectionally.

Examples of the disclosure include:
1. A valve stent used safely, comprising:
   a tubular supporting net frame, and
   a flared section connected to a corresponding end of the supporting net frame;
   wherein the flared section is connected to all end nodes located at a corresponding side of the supporting net frame.
2. The valve stent used safely according to example 1, wherein the flared section is located at a precedent release end of the valve stent.
3. The valve stent used safely according to example 1 or 2, wherein an outer rim of the flared section is formed by a plurality of curved supporting bars, and the end nodes of the supporting net frame corresponding to the positions of the supporting bars are all connected to the supporting bars.
4. The valve stent used safely according to example 3, wherein, the end nodes intersect the supporting bars, or are connected with the supporting bars tangentially and intersectingly by guiding bars.
5. The valve stent used safely according to example 4, wherein each of the guiding bars gradually deviates away from the supporting net frame along an extending path from a corresponding end node to a corresponding supporting bar.
6. The valve stent used safely according to example 5, wherein, an included angle formed by a line connecting two ends of the extending path and an axis of the valve stent is in a range of 0-70 degrees.
7. The valve stent used safely according to example 5, wherein an included angle formed by a line connecting two ends of the extending path and an axis of the valve stent is in a range of 20-60 degrees.
8. The valve stent used safely according to example 6, wherein, an intersection angle formed between the guiding bar and the supporting bar at the junction is an acute angle.
9. The valve stent used safely according to example 1 or 2, wherein a section of the supporting net frame is a transition section, and a ratio of an axial length of the transition section before being compressed to an axial length of the transition section after being compressed is 1.
10. A valve replacement device, comprising:
   a valve stent of any one of examples 1-9; and
   a prosthesis valve fixed inside the supporting net frame.

## Claims

1. A valve stent, comprising:
a tubular supporting net frame (12), and
a flared section (1) connected to a corresponding end of the supporting net frame (12);
wherein the flared section (1) is connected to all end nodes (9) located at a corresponding side of the supporting net frame (12),
**characterized in**
**that** a section of the supporting net frame (12) is a transition section (3), and a ratio of an axial length of the transition section (3) before being compressed to an axial length of the transition section (3) after being compressed is 1.

2. The valve stent according to claim 1, wherein the axial length of the transition section (3) equals at least 40% of a total length of the supporting net frame.

3. The valve stent according to claim 1, wherein the axial length of the transition section (3) equals 40%-90% of the total length of the supporting net frame.

4. The valve stent according to claim 1, wherein the axial length of the transition section (3) equals 50%-80% of the total length of the supporting net frame.

5. The valve stent according to claim 1, wherein the flared section (1) is located at a precedent release end of the valve stent.

6. The valve stent according to claim 1 or 5, wherein an outer rim of the flared section is formed by a plurality of curved supporting bars (6), and the end nodes (9) of the supporting net frame (12) corresponding to the positions of the supporting bars (6) are all connected to the supporting bars (6).

7. The valve stent according to claim 6, wherein, the end nodes (9) intersect the supporting bars (6) or are connected with the supporting bars (6) tangentially and intersectingly by guiding bars (8).

8. The valve stent according to claim 7, wherein each of the guiding bars (8) gradually deviates away from the supporting net frame (12) along an extending path from a corresponding end node (9) to a corresponding supporting bar (6).

9. The valve stent according to claim 8, wherein, an included angle formed by a line connecting two ends of the extending path and an axis of the valve stent is in a range of 0-70 degrees.

10. The valve stent according to claim 8, wherein an included angle formed by a line connecting two ends of the extending path and an axis of the valve stent is in a range of 20-60 degrees.

11. The valve stent according to claim 9, wherein, an intersection angle formed between the guiding bar (8) and the supporting bar (6) at the junction is an acute angle.

12. The valve stent according to claim 1, wherein the transition section (3) consists of a plurality of straight rods (7) extending along an axial direction of the valve stent and evenly distributed along a circumference of the valve stent.

13. The valve stent according to claim 12, wherein the supporting net frame (12) further comprises a first grid portion (4) and a second grid portion (2) respectively connected with two opposite ends of the transition section (3), the first grid portion (4) and the second grid portion (2) respectively consist of continuous rhombic structures, and two opposite ends of each straight rod (7) are respectively connected with a corresponding rhombic vertice of a rhombic unit.

14. The valve stent according to claim 13, wherein the straight rods (7) of the transition section (3) and rims of adjacent rhombic units cooperatively form hexagons, and interior angles of the hexagons are all obtuse angles.

15. A valve replacement device, comprising:
a valve stent of any one of claims 1-14; and
a prosthesis valve fixed inside the supporting net frame (12).
